# EUROPEAN PATENT APPLICATION

(11) **EP 1 376 021 A1**
(43) Date of publication of application: **02.01.2004**
(21) Application number: 01273204.6
(22) Date of filing: 28.12.2001
(51) Int. Cl.: F24F 7/06, A61L 9/18, A61L 2/08, F24F 3/16

(54) **AIR FEED SYSTEM, AIR DEZYMOTIZING METHOD, AND SYSTEM AND METHOD FOR TRANSFER**

(30) Priority: 11.01.2001 JP 2001004068; 05.12.2001 JP 2001372045
(71) Applicant: Mitsubishi Heavy Industries, Ltd., Tokyo 100-8315 (JP)
(72) Inventor: OONO, Yukihiko, c/o KOBE Shipyard & Mach. Works, Kobe-shi, Hyogo 652-8585 (JP)
(74) Representative: HOFFMANN - EITLE
(86) International application number: PCT/JP2001/011624
(87) International publication number: WO 2002/055940

(57) **Abstract**

In an air supplying system (1), a filter (6) for removing dust particles from air is provided at some point in an air supply duct (4) that connects an air supplying section (2) to a specific area (C) . A sterilizing section (10) including an electron beam irradiation unit (11) is provided between the air supplying section (2) and the filter (6) . In an air sterilizing method of the present invention, dust particles in the air are removed by the filter (6) at some point in the air supply duct (4) which connects the air supplying section (2) to the specific area (C), and electron beams are irradiated to the air passing through the air supply duct (4) between the air supplying section (2) and the filter (6).

## Description

### TECHNICAL FIELD

The present invention relates to an air supplying system, an air sterilizing method, a conveyance system, and a conveyance method.

### BACKGROUND ART

Generally, facilities like hospitals, factories that produce medical products, food products, semiconductors or biotechnology related products need highly clean air containing no dust particles. For this reason, in these facilities, an air supplying system is formed by connecting a specific area and an air supplying section including a blower and the like via an air supply duct. In this type of air supplying system, a HEPA filter and the like is provided in the air supply duct. The dust particles discharged from the air supplying section and passing through the duct are prevented by the HEPA filter from being transported to the specific area.

However, the conventional air supplying system has a problem as explained below. There are microscopic fungi (virus, fungi, microorganisms) sticking to the dust particles in the air. So if the filter like HEPA filter catches the dust particles in the air, the fungi which stick to the HEPA filter disadvantageously grow on the filter. These fungi are disadvantageously transported to the downstream, to the specific area.

In addition to dust, the air contains microscopic fungi. There is a problem that these fungi may pass through the filter or pinholes and reach to the specific area. To solve these problems, a filter with finer mesh can be used, but using finer mesh makes a greater pressure loss inside the air supply duct. Moreover, the filter easily gets clogged and further increases the pressure loss. As the result, the filter needs to be replaced often and ends up with an increase of a running cost of the air supplying system.

Recently, terrorismby anthrax broke out in the United States and other places. The terrorism has become an international problem. In these terrorist's attacks, many people died or got injured by infection of anthrax planted in the posted mail when they handled or opened the mail. The mail service is disturbed by the excessive awareness to the mail, and people's peaceful life is disturbed. Currently, clerks at the post office use gloves when they handle the postage because the conventional conveyance system does not give them protection against pollution. However, using gloves do not solve the problem fundamentally and prompt action is desired to the industrial world against the aggravated terrorism.

The object of the present invention is to provide an air supplying system and an air sterilizing method that reliably and effectively prevent an invasion of fungi to the specific areas. Further, this invention relates to the conveyance system and method, and more precisely, it is developed for the pollution abatement of mails, parcels from delivering service, or other delivery matters in anthrax terrorism explained above or other similar cases. Another object of the present invention is to provide a conveyance system and method of effectively sterilizing delivery matters while the matters are conveyed without any trouble.

### DISCLOSURE OF THE INVENTION

The air supplying system according to the present invention sends out air from an air supplying section, and removes dust particles in the air to supply the air containing no dust particles to a specific area. The air supplying system comprises an air supply duct which connects the air supply section to the specific area, a filter which is provided at some point in the air supply duct and removes the dust particles in the air, and a sterilizing section which includes an electron beam irradiation unit that irradiates electron beams to the air passing through the air supply duct, and which is provided between the air supplying section and the filter, or between the filter and the specific section.

This air supplying system is suitable for facilities like hospitals, factories that produce medical products, food products, semiconductors or biotechnology related products or for the places where clean air is needed. The air supplying system is equipped with the air supplying section including a blower. The air supplying section is connected to the specific area of an applied facility, via the air supply duct. The air supply duct loads the filter to remove the dust particles in the air sent out from the air supplying section. Also, the sterilizing section is provided between the air supplying section and the filter, or the filter and the specific area.

In the sterilizing section, the electron beam irradiation unit is provided to irradiate electron beams to the air passing through the air supply duct. For the electron beam irradiation unit, electron beam energy, electric current value and other values are determined by taking into account a radiation resistance of the major fungi or fungi that must be removed from the air surrounding the air supplying system and the amount of supplying air (width and height of the air supply duct) from the air supplying section.

When the air supplying system configured above is provided with the sterilizing section between the air supplying section and the filter, the electron beams are irradiated to the air sent out from the air supplying section and passing through the air supply duct in the sterilizing section positioned upstream of the filter. With the beams, most of the fungi (virus, fungi, microorganisms) in the air are killed before they reach the filter. These killed fungi alone or with the dust particles are caught by the filter. Even if the fungi pass through the filter and reach up to the specific area, these fungi are already destroyed by the irradiated electron beams. As the result, this air supplying system prevents invasion of fungi to the specific area reliably and efficiently. Also, the fungi caught by the filter or the fungi stuck to the dust particles that are caught by the filter are already killed. Consequently, the fungi can be prevented from growing on the filter. Therefore, it is possible to reduce the frequency of replacing the filter.

On the other hand, when the sterilizing section is provided between the filter and the specific area of the air supplying system in the air supply system, the air sent out from the air supplying section passes the filter first. Accordingly, the dust particles in the air passing through the air supply duct are caught by the filter. Then, the air from which the dust particles are sufficiently removed is irradiated with the electron beams in the sterilizing section that is positioned downstream of the filter. With this irradiation, most of the fungi (virus, fungi, microorganisms) that are not caught by the filter are killed before entering the specific section. As the result, this air supplying system prevents invasion of fungi to the specific area reliably and efficiently. This configuration is especially suitable for the places where many dust particles are contained in the surrounding air, the air supplying section takes in the air only from outside, and the air from the specific area is discharged to outside.

It is favorable to provide a plurality of the electron beam irradiation units and dispose these units facing each other so that the air supply duct is disposed between the facing units.

By using the configuration above, even if a distance between the electron beam irradiation units is elongated, or if the width and height of the air supply duct are enlarged, it is still possible to irradiate the air passing through the air supply duct with the electron beams perfectly. Therefore, this configuration is especially favorable to the case where a cross section of the duct needs tobe increased in order to supply a large amount of air to the specific area.

Further, it is favorable to provide a bent on the air supply duct, position the filter near the bent, and place the electron beam irradiation unit near the bent so that the electron beams can be irradiated to the surface of the filter.

With such a configuration, the filter is placed downstream of the bent and the electron beam irradiation unit is placed, for instance, upstream of the filter behind the bent. Accordingly, the electron beams emitted from the electron beam irradiation unit are irradiated to the air passing through the bent of the air supply duct and are also irradiated to the surface of the filter. As a result, the air supplying system can kill the fungi in the air, those caught by the filter, and those stuck to the dust particles caught by the filter. Therefore, by using this air supplying system, invasion of the fungi to the specific area is reliably and effectively prevented. Also, it is possible to further reduce the frequency of replacing the filter because the fungi are prevented from growing on the filter.

Further, it is preferable that the electron beam irradiation unit is disposed so that the electron beams reflected by the internal surface of the air supply duct can reach the surface of the filter.

In this case, the electron beam irradiation unit is disposed in the vicinity on the upstream or the downstream of the filter. Accordingly, the electron beams emitted from the unit are irradiated to the air passing through the air supply duct and are also reflected by the internal surface of the air supply duct to reach the surface of the filter. As a result, this air supplying system can kill the fungi in the air, those caught by the filter, and even those stuck to the dust particles caught by the filter. Therefore, by using this air supplying system, invasion of the fungi to the specific area is reliably and effectively prevented. In addition, the fungi can be prevented from growing on the filter. Thus, it is possible to further reduce the frequency of replacing the filter.

Moreover, it is favorable to have a fluorescent body in the air supply duct which emits light by a stimulus of the electronbeams emitted from the electronbeamirradiation unit.

The fluorescent body is disposed in the air supply duct based on the configuration so the fluorescent body faces the electron beam irradiation unit. The electron beams emitted from the electron beam irradiation unit are irradiated to the air passing through the air supply duct and are also let in the fluorescent body. Accordingly, the fluorescent body generates ultraviolet rays by a stimulus of the electron beams, and by the ultraviolet rays, the specific fungi in the air passing through the air supply duct are killed. By using the air supplying system, the ability to sterilize the fungi by the electron beam irradiation unit is further improved.

In this case, the fluorescent body is preferably disposed on the bottom of the internal side and the internal side faces of the air supply duct.

By employing such a configuration, it is possible to further increase the amount of ultraviolet rays emitted from the fluorescent bodies.

The air supplying system according to the present invention can employ any device as the air supplying section which takes in the air from outside to mix it with the air sucked from the specific area and sends out the mixed air to the air supply duct. It is also possible to employ a device, as the air supplying section, which takes in the air only from the specific area, or to employ a device which takes in the air only from the outside and discharges the air in the specific area to outside.

The air sterilizing method according to the present invention comprises steps as follows. That is, the method comprises sending out the air from an air supply section, and sterilizing the air by removing dust particles in the air when the air is supplied from the air supplying section to the specific area. The method also comprises removing the dust particles in the air by the filter provided at some point in the air supply duct connecting the air supplying section to the specific area, and irradiating the electron beams to the air passing through the air supply duct between the air supply section and the filter or between the filter and the specific area.

It is favorable to irradiate the air in the supplying duct with the electron beams from positions opposite to each other where the air supply duct is disposed between the positions.

Further, it is favorable to provide a bent on the air supply duct, dispose the filter near the bent, and irradiate the surface of the filter with the electron beams.

Furthermore, it is favorable to let the electron beams reflected by the internal surface of the air supply duct, into the surface of the filter.

Furthermore, it is favorable to dispose a fluorescent body which emits light by a stimulus of the electron beams in the air supply duct, and to irradiate the beams to the fluorescent body.

In this case, the fluorescent body is preferably disposed on the bottom of the internal side and the internal side faces of the air supply duct.

In this invention, delivery matters like mails are conveyed by the conveying units like a conveyor, and at the same time, the delivery matters are irradiated with the electron beams through the electron beam irradiation unit which is provided near the conveying unit. Accordingly, the safety of persons who handle the mails are ensured by sterilizing fungi that may be enclosed in the mails. In addition, efficiency of the work can be achieved by carrying out the sterilizing process during the conveyance. The delivery matters include box type small parcels as well as thin-structured mails. The conveying unit includes a belt conveyor or a roller conveyer.

The conveyance system of the present invention comprises a conveying unit including a conveyer or other units which convey delivery matters such as mails and deliveryparcels, and an electron beam irradiation unit which is disposed near the conveying unit and irradiates the electron beams to the delivery matters during conveying process.

In this invention, the delivery matters like mails are conveyed by the conveying unit and at the same time the electron beams are irradiated to the mails using the electron beam irradiation unit disposed near the conveying unit. Therefore, the safety of persons who handle the mails can be ensured by sterilizing fungi enclosed in the mails. It is advantage that the sterilizing process is carried out during the conveying process and the efficiency of the work is achieved. The case of "during the conveying process" includes a case where the electron beams are irradiated to the delivery matters when they are temporarily stopped, in addition to the case where the beams are irradiated while the delivery matters are being conveyed.

The conveyance system of the present invention includes a flat part comprising a belt part, a roller part, a plate shaped part, and other parts. The system also includes the conveying unit which conveys thin delivery matters such as thin-structured mails and sealed letters with each front face or back face of the matters down on the flat part, and the electron beam irradiation unit which is provided so that the direction of irradiating the beams is substantially perpendicular to the flat part, and which irradiates the electron beams to the delivery matters during the conveying process thereof.

In this invention, the thin delivery matters are irradiated with the electron beams through the electron beam irradiation unit in which the direction of irradiating the beams is substantially perpendicular to the flat part of the conveying unit while the matters are laid on the flat part and conveyed. Accordingly, the sterilization effect is enhanced because the electron beams can be irradiated in a direction substantially perpendicular to the delivery matters. In addition, it is advantage that the sterilizing process is efficiently carried out because the thin delivery matters are irradiated with the electron beams during the conveying process.

The conveyance system according to the present invention includes an electron beam irradiation unit which irradiates electron beams, a conveying unit which conveys thin delivery matters such as thin-structured mails and sealed letters, and a delivery matter sending unit. The deliverymatters sendingunit sends the thindeliverymatters into an area where the beams are irradiated by the electron beam irradiation unit, in such a manner that each front face or back face of the matters is directed substantially perpendicularly to the direction of irradiating the electron beams.

In this invention, the thin delivery matters are sent into an area where the electron beams are irradiated by the electron beam irradiation unit in such a manner that each front face or back face of the matters is directed to a direction substantially perpendicular to the beam irradiating direction, and the matters are actually irradiated with the beams. Therefore, the matters are sterilized effectively.

This conveyance system according to the present invention, based on the above-mentioned conveying system, comprises a pair of electron beam irradiation units positioned opposite to each other in a vertical direction or a horizontal direction with respect to the conveying unit.

In this invention, by using the paired electron beam irradiation units provided in the above manner, each of the delivery matters is irradiated with the electron beams from both side of the matter, and therefore the beams reach the matter deeper inside and can sterilize it more effectively.

In this conveyance system according to the present invention based on the above-mentioned conveying system, a conveying unit includes a spreading unit which spreads the delivery matters so that they will not overlap in an area where the electron beams are irradiated.

In this invention, the conveying unit is provided with the spreading unit to spread the delivery matters so as not to be overlapped in the area where the beams are irradiated. Therefore, the electron beams are irradiated evenly to each face of the delivery matters. This makes it possible to reliably sterilize the matters.

The conveyance method according to the present invention includes a step of conveying thin delivery matters such as thin-structured mails and sealed letters with each front face or back face of the matters down on a flat part. The flat part is formed of a belt part, a roller part, a plate part, or some other parts. The method also includes a step of irradiating electron beams to each front face or back face of the thin delivery matters substantially perpendicularly by the electron beamirradiation unit during the conveying process.

In this invention, each front face or back face of the thin delivery matters is irradiated with the electron beams by the electron beam irradiation unit, and the beam irradiating direction is directed to the flat part of the conveying unit on which the matters are conveyed. Thus, each face of the thin delivery matters is sterilized effectively during the conveying process. The beams may be irradiated to the matters while the conveying unit is operating or when the conveying unit is temporarily stopped.

The conveyance method according to the present invention includes a step of conveying thin delivery matters such as thin-structured mails and sealed letters, and a step of sending the matters into an area where electron beams are irradiated by an electron beam irradiation unit in such a manner that each front face or back face of the matters is directed substantially perpendicularly to the beam irradiating direction.

In this invention, the thin delivery matters are sent into the area where the electron beams are irradiated by the electron beam irradiation unit in such a manner that each front face or back face of the matters is directed substantially perpendicularly to the beam irradiating direction, and the matters are irradiated with the beams. Thus, the delivery matters are sterilized effectively.

The conveyance method according to the present invention, based on the above-mentioned conveying method, further includes a step of spreading thin delivery matters on a flat part so that the matters do not overlap in an area where electron beams are irradiated.

In this invention, the delivery matters are spread on the flat part of the conveying unit so as not to overlap in the area where electron beams are irradiated. Therefore, the electron beams are irradiated evenly onto each face of the thin delivery matters. This makes it possible to reliably sterilize the matters.

### BRIEF DESCRIPTION OF THE DRAWINGS

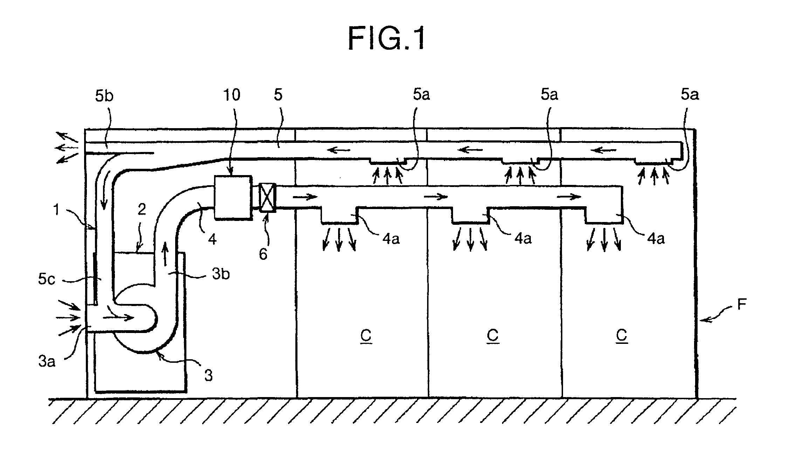

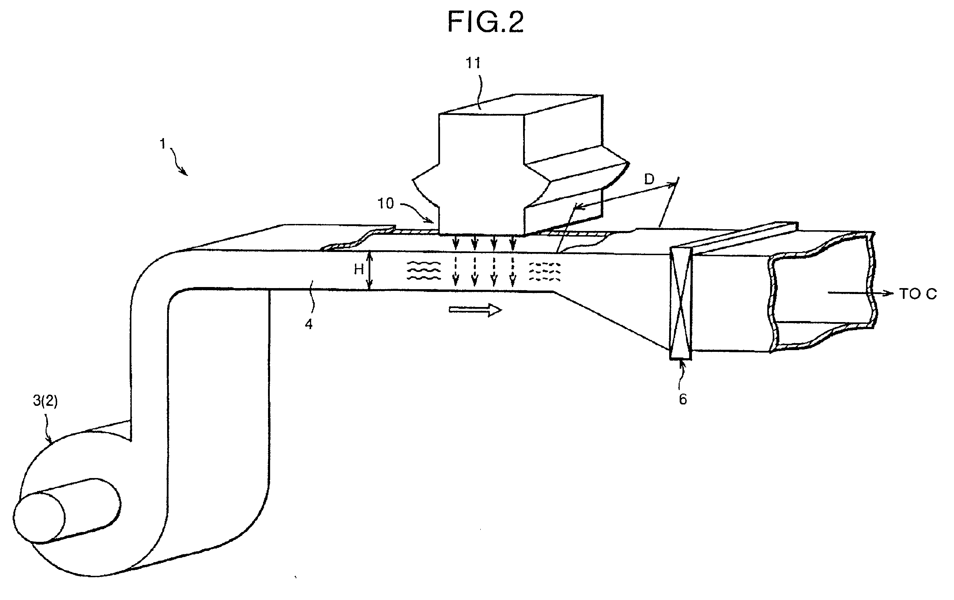

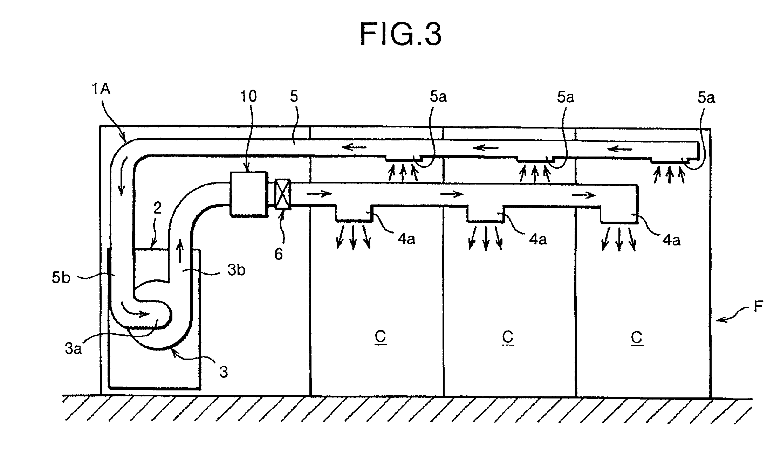

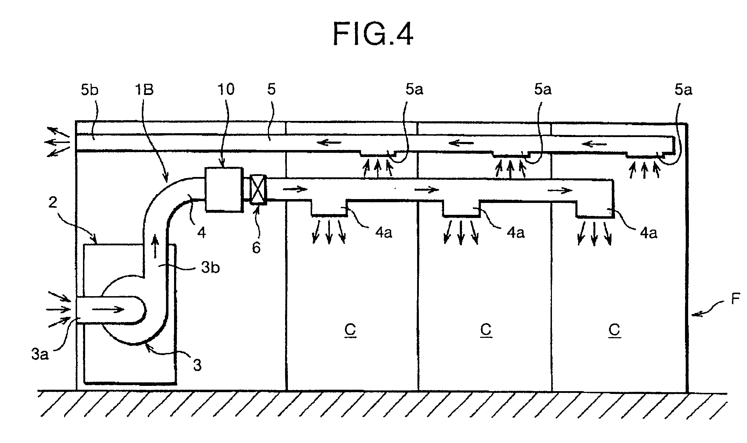

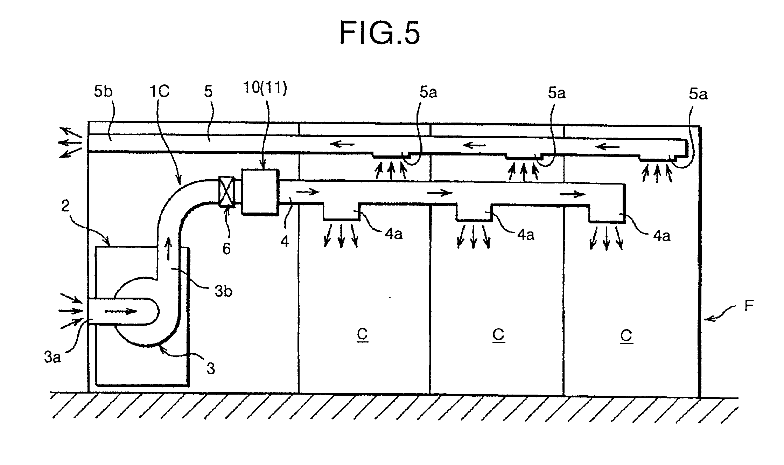

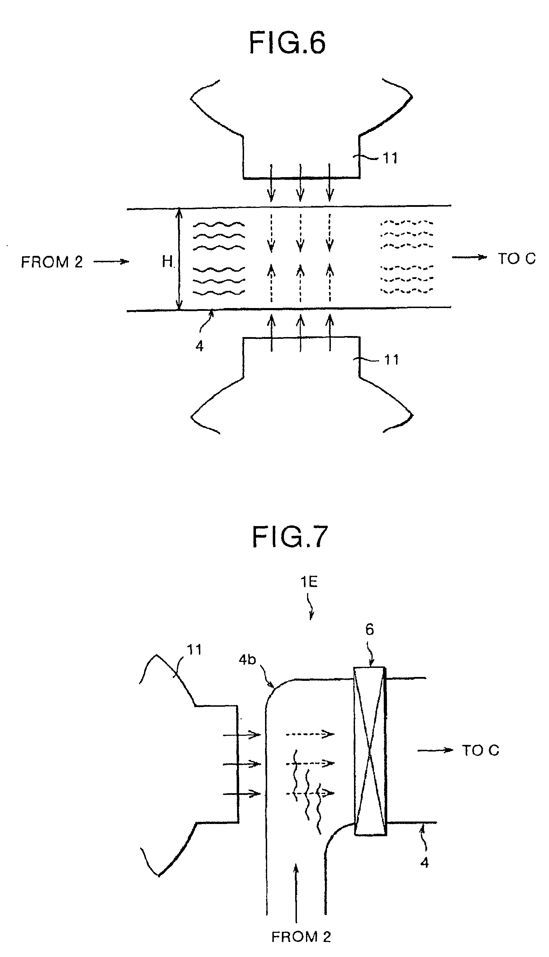

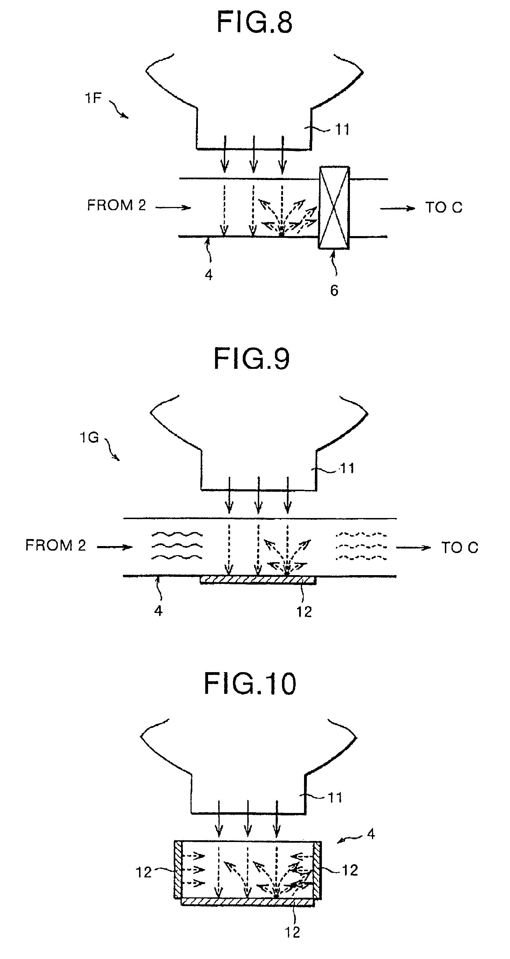

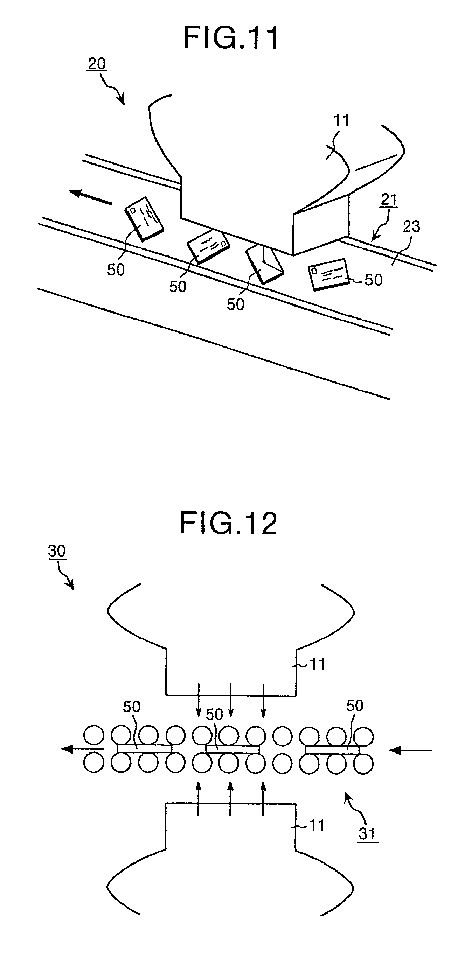

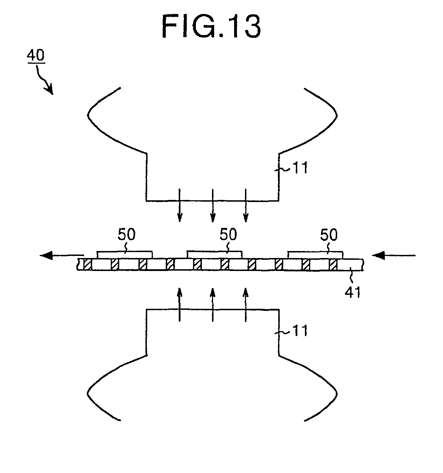

Fig. 1 is a schematic diagram showing a first embodiment of the air supplying system according to the present invention, Fig. 2 is a perspective view showing a key section of the air supplying system in the Fig. 1, Fig.3 is a schematic diagram showing modification of the air supplying system according to the first embodiment, Fig.4 is a schematic diagram showing another modification of the air supplying system according to the first embodiment, Fig.5 is a schematic diagram showing still another modification of the air supplying systemaccording to the first embodiment, Fig.6 is a schematic diagram showing a second embodiment of the air supplying system according to the present invention, Fig.7 is a schematic diagram showing a third embodiment of the air supplying system according to the present invention, Fig.8 is a schematic diagram showing a forth embodiment of the air supplying system according to the present invention, Fig.9 is a schematic diagram showing a fifth embodiment of the air supplying system according to the present invention, Fig.10 is a schematic diagram showing modification of the air supplying system according to the fifth embodiment, Fig. 11 is a perspective view showing a key section of the conveyance system according to a sixth embodiment of the present invention, Fig. 12 is a side view showing a key section of the conveyance system according to a seventh embodiment of the present invention, and Fig.13 is a side view showing modification of the conveyance system according to the seventh embodiment.

### BEST MODE FOR CARRYING OUT THE INVENTION

Embodiments of the air supplying system and the air sterilizing method according to the present invention will be described in detail with reference to the accompanying drawings. It should be noted here that the present invention is not limited by these embodiments.

### First embodiment:

Fig. 1 is a schematic diagram showing the first embodiment of the air supplying system according to the present invention. The air supplying system 1 shown in the Fig. 1 is favorable to be used in the facilities like hospitals, factories that produce medical products, food products, semiconductors or biotechnology related products where clean air without dust particles are needed. The system 1 supplies the clean air without dust particles to a specific area C (in this embodiment, there are plurality of areas) of a facility F.

As shown in the Fig. 1, the air supplying system 1 is provided with an air supplying section 2. The air supplying section 2 has a blower 3 that takes in the outer air from a suction port 3a and discharges from a discharge port 3b. An air conditioning unit (not shown in the figure) which includes a heat exchanger and a compressor is provided in the air supplying system 2 as required. The discharge port 3b of the blower 3 which is provided in the air supplying section 2 is connected to the air supply duct 4. The air supply duct 4 is installed so as to pass through each specific area C as shown in the Fig. 1. The air supply duct 4 has an air supply port 4a corresponding to each specific area C. By the duct, the air supplying system 2 is connected to each specific area C of the facility via the air supply duct 4.

The air supplying system 1 includes an air discharge duct 5. The duct 5 is installed so as to pass through each specific area C. The duct 5 has an air suction port 5a corresponding to each specific area C. The end of the duct 5 is branched into two directions . As shown in Fig. 1, one end 5b is open to outside, and the other end 5c is connected to the suction port 3a of the blower 3 in the air supplying section 2. When the blower 3 is activated, the air from outside (fresh air) and the air sucked from the specific areas C (circulating air) via the air discharge duct 5 are mixed and balanced in a specific ratio (for example, 43:57), and the air is provided from the air supplying section 2 to the specific areas C. Of the air which is sucked from the specific areas C, part of the air which is not reused as the circulating air is discharged to outside from the end 5b of the air discharge duct 5.

As seen from Fig. 1, a filter 6 is provided at some midpoint on the air supply duct 4 to remove the dust particles from the air supplied from the air supplying section 2 . Dust particles in the air which is discharged from the air supplying section 2 and passes through the air supply duct 4, is mechanically caught and removed by the filter 6 before the air is inducted into the specific area C. The filter 6 can be various types of HEPA filter. In addition, the air supplying system 1 has a sterilizing section 10 provided between the air supplying section 2 and the filter 6.

As seen in Fig. 2, the sterilizing section 10 has an electron beam irradiation unit 11. The electron beam irradiation unit comprises an electronic gun and an electric power supply, and has ability to irradiate electron beams. In the air supplying system 1, the air, which is discharged from the air supplying section 2 and passes through the air supply duct 4, is sterilized by the electron beams irradiated from the electron beam irradiation unit 11 in the sterilizing section 10 that is positioned upstream of the filter 6. Therefore, most of the fungi (virus, fungi, microorganisms) that are floating in the air are killed before they reach the filter 6. The killed fungi alone or with the dust particles are collected by the filter 6.

Even if small fungi pass through the filter 6 and reach the specific area C, the fungi are already killed by the electron beams irradiated at the sterilizing section 10. So, according to the air supplying system 1, invasion of the fungi to the specific area C is reliably and effectively prevented. Also, the fungi that are caught by the filter 6 or the fungi that are stuck to the dust particles caught by the filter 6 are also killed, so such fungi will not grow on the filter 6. Therefore, it is possible to replace the filter far less than before.

For the electron beam irradiation unit 11, the electron beam energy, current value, and other values are determined based on the radiation resistance of major fungi or fungi that must be removed in the surrounding air around the supplying system and the amount of air supplied (width and height of an air duct) from the air supplying section. That is, when the major fungi have been irradiated with y ray by the dose (kGy) shown in Table 1, the fungi can be decreased by 10⁶ Colony Foaming Unit (CFU). For the electron beam, the current value that is to be supplied to the electron beam irradiation unit 11 is determined by dividing the value on the right hand side of Table 1 by 5 and multiplying by 80 (mmA).

**TABLE 1**

| Fungi | Dose (kGy) needed to lower by 10⁶ |
|---|---|
| E.coli(Escherichia coli) | < 0.5~3 |
| B.cereus(spore) | 20-30 |
| B.pumilus(spore) | 1-30 |
| B.subtilis(spore) | 10-20 |
| Asp.niger(Black-koji mold) | 3-5 |

Table 2 shows examples of the height of the air supply duct 4 that provides the amount of irradiation of the electron beam equivalent to the same sterilizing ability. As shown in Fig. 2, for example, the electron beam energy is set to 120kV, width D of the air supply duct 4 (see Fig. 2) is set to 40cm, and height H (see Fig. 2) is set to 14cm, and the current value of the electron beam energy is set to 80mmA, then the electron beam irradiation unit 11 have a sterilizing ability corresponding to 2000m³/h of air flow.

**TABLE 2**

| Electron beam energy (kGy) | Height (cm) of airsupply duct |
|---|---|
| 150 | 20.2 |
| 140 | 18.2 |
| 130 | 16.1 |
| 120 | 14.0 |
| 110 | 12.4 |
| 100 | 10.5 |
| 95 | 9.7 |
| 90 | 8.8 |
| 85 | 7.4 |
| 80 | 6.6 |

In the air supplying system 1, the air supplying section 2 has such a configuration that the air from the outside and the air sucked from the specific areas C are mixed and discharged from to the air supply duct 4. However, the present invention is not limited by this configuration. The air may be taken only from the specific area C like an air supplying system 1A show in Fig. 3. In this case, the end 5b of the air discharge duct 5 may be directly connected to the suction port 3a of the blower 3 and the duct end does not need to be branched.

Like the air supply system 1B in the Fig. 4, the outside air (fresh air) and the air in the specific area C may be fully exchanged. In this case, the suction port 3a of the blower 3 included in the air supplying section 2 is connected only to outside. The end 5b of the air discharge duct 5 is only connected to the outside. Accordingly, the air supplying section 2 takes in only the outside air and the air sucked from the specific area C is discharged without recycling as circulating air.

Fig.5 shows the modification of the air supply system according to the first embodiment. An air supplying system 1C in this figure is different from the air supplying system 1B in that the sterilizing section 10 including the electron beam irradiation unit 11 is provided between the filter 6 and the specific area C but not between the air supply section 2 and the filter 6. In the system 1C constructed in the above manner, the air which is discharged from the air supplying section 2 to the air discharge duct 4 first passes the filter 6. So the dust particles in the air are caught by the filter 6.

Passing the filter 6, the cleaned air without dust particles are irradiated with the electron beam at the sterilizing section 10 which is positioned downstream of the filter 6. Therefore, most of the fungi (virus, fungi, microorganisms) that are not caught by the filter are killed before they reach the specific area C. So, according to the air supplying system 1C, invasion of the fungi to the specific area C is reliably and effectively prevented. This construction is especially appropriate for the place where the surrounding air contains many dust particles and the air is inducted by the air supplying section 2 only from the outside while the air in the specific area C is discharged outside.

It is possible to provide the sterilizing section 10 including the electron beam irradiation unit 11 to the place between the filter 6 and the specific area C for the air supplying system 1 or 1B (not shown in the figure). In these cases, the system 1 employs the configuration, as the air supplying section 2, that mixes a fresh air taken from the outside with the air sucked from the specific area C and discharges the mixed air to the air supply duct 4 as the air supplying section 2, and the system 1B employs the configuration, as the air supplying section 2, that takes in the air only from the specific area C.

### Second embodiment:

By referring to Fig.6, the second embodiment of the air supplying system and the air sterilizing method of present invention is explained below. The same reference numerals are assigned to those corresponding to the components in the first embodiment, and explanations of the components are omitted.

An air supply system 1D shown in Fig. 6 has a plurality of the electron beam irradiation units 11. The electron beam irradiation units 11 are disposed in the upper side and the lower side of the air supply duct 4 so as to sandwich the air supply duct 4, and the electron beams are irradiated to the air passing the air supply duct 4 from the disposed positions opposite to each other. By employing such a construction, it is still possible to irradiate the electron beams reliably to the air passing through the air supply duct 4 even the space between the electron beam irradiation units 11 or the height H of the duct 4, is increased. The electron beam irradiation units 11 can be also disposed on both the right and left sides of the air supply duct 4 so as to be opposite to each other (not shown on the figure). In this case, making the width wider still allows the electron beam to reach the air passing through the air supply duct 4. Thus, the construction is particularly favorable to a case where a wider cross sectional area of the air supply duct 4 is needed to supply more air to the specific section C.

The filter may be disposed on either the upstream or the downstream of the each electron beam irradiation unit 11. The air supplying section 2 of the air supplying system 1D in the second embodiment can employ a system that takes the air from outside and mixes it with the air from the specific area and the mixed air is discharged from the air supply duct 4. Further, the air supply section 2 employs any system that takes in the air only from the specific area or that takes in the air only from outside to discharge the air of the specific area C to outside.

### Third embodiment:

The third embodiment of the air supplying system and the air sterilizing method in present invention is explained below with reference to Fig.7. The same reference numerals are assigned to those corresponding to the components in the first embodiment, and explanations of the components are omitted.

The system 1E shown in Fig. 7 has a bent 4b formed at the air supply duct 4, and the filter 6 is positioned downstream of the bent 4b. The electron beam irradiation unit 11 is positioned behind the bent 4b which is provided upstream of the filter 6 so as to irradiate the electron beam to the surface of the filter 6. By forming the bent 4b at the air supply duct 4, disposing the filter near the bent 4b, and positioning the electron beam irradiation unit 11 near the bent 4b, the electron beam generated from the electron beam irradiation unit 11 can be irradiated to the air passing the air supply duct 4 (bent 4b) and to the surface of the filter 6.

As the result, the air supplying system 1E makes it possible to kill the fungi caught by the filter or the fungi which are stuck to the dust particles caught by the filter 6, as well as the fungi in the air. According to the air supplying system 1E, invasion of the fungi to the specific area C is reliably and effectively prevented. It is also possible to prevent the filter from growing of the fungi thereon, which makes it possible to reduce the frequency of replacing the filter 6.

Although not shown in the figure, the filter 6 may be disposed on the upstream of the bent 4b and the electron beam irradiation unit 11 may be disposed on the downstream so that the surface of the filter 6 is irradiated with electron beams. The air supplying system 1E in the third embodiment can employ a system, as the air supply section 2, that takes in the air from outside and mixes it with the air from the specific area to discharge the mixed air to the air supply duct 4. Furthermore, any system may be employed as the section 2 if the system takes in the air only from the specific area C or the system takes in the air only from outside to discharge the air from the specific area C to outside.

### Forth embodiment:

The forth embodiment of the air supplying system and the air sterilizing method of present invention is explained below with reference to Fig. 8. The same reference numerals are assigned to those corresponding to the components in the first embodiment, and explanations of the components are omitted.

The system 1F shown in Fig. 8 has the electron beam irradiation unit 11 positioned in the vicinity and upstream of the filter 6. By positioning the unit 11 upstream, the electron beams generatedfrom the electron beam irradiation unit 11 are irradiated to the air passing through the air supply duct 4 and are reflected by the internal side (bottom) of the duct 4. Then, the reflected beams enter the surface of the filter 6. As a result, the air supplying system 1F makes it possible to kill the fungi caught by the filter 6 and the fungi which are stuck to the dust particles caught by the filter 6, as well as the fungi in the air. According to the air supplying system 1F, invasion of the fungi to the specific area C is more reliably and effectively prevented. The system also prevents the filter from growing of the fungi thereon, which makes it possible to reduce the frequency of replacing the filter.

Although not shown in the figure, the electron beam irradiation unit 11 may be disposed in the vicinity and downstream of the filter 6. In this case, the reflected electron beam can be let into the surface of the filter 6. The air supplying system 1F in the forth embodiment can also employ any system, as the air supply section 2, that takes in the air from outside and mixes it with the air from the specific area and discharges the mixed air to the air supply duct 4. Furthermore, any system may be employed as the section 2 if the system takes in the air only from the specific area C or the system takes in the air only from outside to discharge the air from the specific area C to outside.

### Fifth embodiment:

The fifth embodiment of the air supplying system and the air sterilizing method of the present invention is explainedbelowwith reference to Fig. 9. The same reference numerals are assigned to those corresponding to the components in the first embodiment, and explanations of the components are omitted.

An air supply system 1G in Fig. 9 has a fluorescent body 12 disposed inside the air supply duct 4. The fluorescent body 12 emits light by a stimulus of the electron beams generated from the electron beam irradiation unit 11. Inside the air supply duct 4, the fluorescent body 12 is positioned to face the electron beam irradiation unit 11. The electron beams that are generated by the electron beam irradiation unit 11 are irradiated to the air passing through the air supply duct 4 and are also let into the fluorescent body 12. By the stimulus of the electron beams, the fluorescent body 12 generates ultraviolet rays, and the ultraviolet rays are possible to kill predetermined fungi in the air passing through the air supply duct 4.

As explained above, according to the air supplying system 1F, the ability to sterilize the fungi by the electron beam irradiation unit 11 is further improved. As shown in Fig. 10, it is preferable to dispose the fluorescent bodies 12 on the bottom of the internal side and the internal sides of the air supply duct 4 . By placing them in such a manner, the amount of the ultraviolet rays generated from the fluorescent bodies 12 increases.

### Sixth embodiment:

Fig. 11 is a perspective view showing the key section of a conveyance system according to the sixth embodiment. In this figure, the same reference numerals are assigned to those corresponding to the components in the first embodiment, and explanations of the components are omitted. The conveyance system 20 comprises an electron beam irradiation unit 11 and a belt conveyer 21. The conveyer 21 forms a part of an automatic sorting line which conveys delivery matters 50 and sorts the matters automatically, and is disposed upstream of the line. The conveyer 21 has a broad flat part 23 and conveys the matters 50 to a predetermined position by putting the matters 50 on this flat part 23 . The unit 11 is situated above the belt conveyer 21 and a radiation aperture of the electron beams is disposed so as to be perpendicular to the flat part 23 of the conveyer 21. The radiation aperture of the beam has substantially the same width as the flat part 23 so that the unit 11 irradiates the electron beams from the aperture evenly over the flat part 23 passing below the unit 11. The delivery matters 50 are mainly thin-structured mails like sealed letters and are spread over the flat part 23 of the conveyer 21 with each front face or back face of the mails down on the flat part 23.

In the sixth embodiment, the delivery matters 50 are spread over the flat part 23 of the conveyer 21 and successively pass below the radiation aperture of the unit 11 as the flat part 23 moves. The delivery matters 50 are sterilized by the electron beams irradiated from the unit 11 and conveyed to the downstream, to an automatic sorting machine (not shown on the figure). If any of the delivery matters 50 has anthrax enclosed, it is promptly and safely dealt with on the conveying line.

According to the sixth embodiment, the electronic beam irradiation unit 11 is disposed upstream of the conveyer 21 and irradiates the electron beams to the delivery matters 50 when the matters 50 pass by. So the sterilizing process can be done without stopping conveying process. Also, the sterilizing process of the delivery matters 50 is normally and promptly carried out without any manpower required so it is highly secured and does not stop the delivery and collection service. Besides, the beams are irradiated from immediately above the delivery matters 50, so the delivery matters 50, especially the thin delivery matters 50 can be sterilized effectively because the area where the beams are irradiated increases.

In this sixth embodiment, the object for sterilizing is the thin delivery matters 50, but it may be box shaped parcels. In that case, it is desired to enforce the efficiency of the sterilization by increasing the output level of the electron beams to reach the electron beams to the delivery matters 50 deeper inside.

In this sixth embodiment, the unit for conveying the delivery matters is the conveyer 21, but it may be a roller type conveyer, a conveyer formed with coupled plate-like members, or other conveying units which are known to the person skilled in the art. A material of broad flat part 23 is not limited in particular and it may be anything if it will not disturb the irradiation of the beams or the conveying of the delivery matters 50.

In this sixth embodiment, a spreading process which spreads the delivery matters 50 over the flat part 23 of the conveyer 21 to prevent overlap of the matters is not mentioned. But spreading of the delivery matters 50 can be carried out by a method which is publicly known to the person skilled in the art. Accordingly, the delivery matters 50 can be irradiated with the electron beams effectively to improve a sterilization effect. The spreading unit can be a unit that forms the flat part 23 with rubber or other rubbing material and slopes the flat part 23 in the upstream of the electronic beam irradiation unit 11 (not shown in the figure). By the configuration above, the delivery matters 50 in contact with the flat part 23 during conveyance are kept to the positions where they are by the friction of the flat part 23 . The delivery matters 50 that are not in contact with the flat part 23, slide over the other matters 50 because of the slope of the flat part 23 and spread to the other place of the flat part 23. This simple slope spreads the delivery matters 50 effectively over the flat part 23.

### Seventh embodiment:

Fig. 12 is a side view showing the key section of the conveyance system according to the seventh embodiment of this invention. The same reference numerals are assigned to those corresponding to the components in the sixth embodiment, and explanations of the components are omitted. The conveyance system 30 is different from the conveyance system 20 according to the sixth embodiment in that the system 30 is provided with a conveying mechanism 31 having rollers facing the conveying unit for conveying the delivery matters 50, and that a pair of the electron beam irradiation units 11 and 11 are provided on both sides of the conveying unit so as to face each other . The roller type conveying mechanism 31 conveys the delivery matters 50 from the right hand side to the left hand side of Fig. 12.

In the seventh embodiment, the delivery matters 50 are held by the opposite rollers to be discharged to the radiation aperture of the electron beam irradiation unit 11 in such a manner that the matters 50 do not overlap each other. The matters 50 are sterilized with the beams irradiated by the units 11, 11 from both sides. Then, each of the matters 50 is conveyed to the automatic sorting machine (not shown in the figure) placed downstream. The delivery matters 50 are irradiated with the beams from the spaces between the rollers in a direction perpendicular to the front and the back of each matter. Accordingly, the delivery matters 50 with anthrax enclosed are quickly and safely treated on the conveying line with enhanced sterilization effect.

According to the seventh embodiment, the delivery matters 50 are sterilized by irradiating the electron beam perpendicularly to the delivery matters 50 from both sides, which result in improvement of the sterilization. The electron beam in particular transmits the object to be irradiated only up to 0.5mm from its surface by energy of about 300kV, but by irradiating the beam to the object from both sides of the object, sufficient effect can be gained. In addition, because the roller type conveying mechanism 31 is employed, the delivery matters 50 are irradiated from spaces between the rollers, which is advantageous.

In the seventh embodiment, the delivery matters 50 are held by the opposite rollers of the roller type conveying mechanism 31 and are irradiated with the electron beams from the back of the rollers through the spaces between the rollers. It is preferable in the sense the delivery matters 50 are irradiated precisely one by one. However, this configuration of the sterilization process is not limited to the above. A conveying unit with a mesh type or porous type conveyer belt 41 may be used to lay the delivery matters 50 on this belt 41 and dispose the electron beam irradiation units 11 and 11 in a vertical direction of the belt 41 so that the delivery matters 50 are irradiated with the electron beams from both sides of the matters 50 (see Fig. 13). By employing such a configuration, effective sterilizing process becomes possible.

The air supplying system and the air sterilizing method according to the present invention are configured as explained above, and therefore effects as follows can be obtained. The air supplying system according to the present invention is provided with the filter which is provided at some point in the air supply duct connecting the air supply section to the specific area, and which removes dust particles in the air. The sterilizing section including the electron beam irradiation unit is provided between the supplying section and the filter or between the filter and the specific area. The air sterilizing method according to the present invention comprises removing the dust particles in the air by the filter provided at some point in the duct that connects the air supplying section and the specific area, and irradiating the beams to the air passing through the air supply duct between the air supplying section and the filter or between the filter and the specific area. This mechanism prevents invasion of fungi to the specific area reliably and efficiently.

According to the conveyance system of the present invention, the delivery matters like mails are conveyed by the conveying unit like conveyer and at the same time the electron beams are irradiated to the mails using the electron beam irradiation unit disposed near the conveying unit. The safety of persons who handle the delivery matters can be ensured by sterilizing the fungi enclosed in the delivery matters. In addition, it is advantage that efficiency of the sterilization is enhanced because the sterilization procedures are carried out during the conveying process of the matters.

According to the conveyance system of the present invention, the delivery matters like mails are conveyed by the conveying unit like conveyer and at the same time the electron beams are irradiated to the mails using the electron beam irradiation unit disposed near the conveying unit . The safety of persons who handle the delivery matters can be ensured by sterilizing the fungi enclosed in the delivery matters. In addition, it is advantage that efficiency of the sterilization is enhanced because the sterilization procedures are carried out during the conveying process of the matters.

According to the conveyance system of the present invention, the thin delivery matters are conveyed in such a manner that the matters are laid on the flat part of the conveying unit, and at the same time the electron beams are irradiated to the matters through the electron beam irradiation unit. The beam irradiating direction is directed substantially perpendicularly to the flat part to the conveying unit. Accordingly, the electron beams can be irradiated nearly perpendicularly to the thin delivery matters, thus enhancing the sterilization effect. In addition, the delivery matters are irradiated with the electron beams during the conveying process, and therefore the sterilizing process is effectively carried out.

According to the conveyance system of the present invention, the thin delivery matters are sent into the area where the electron beams are irradiated from the electron beam irradiation unit, in such a manner that each front face or back face of the matters is directed substantially perpendicularly to the electron beam irradiating direction, and the matters are actually irradiated with the beams. Thus, the delivery matters can be effectively sterilized.

According to this conveyance system of the present invention, the delivery matters are irradiated with the electron beams from both sides of the matters by the paired electron beam irradiation unit which are disposed opposite to each other in the vertical direction or the horizontal direction with respect to the conveying unit. The electron beams are irradiated more deeply to the matters, which makes it possible to enhance the sterilization effect.

According to the conveyance method of the present invention, the conveying unit is provided with the spreading unit. The spreading unit spreads the delivery matters so as not to overlap in the area where the electron beams are irradiated, and therefore the electron beams can be evenly irradiated to each face of the thin delivery matters. Thus, the delivery matters are reliably sterilized.

According to the conveyance method of the present invention, the thin delivery matters laid on the flat part of the conveying unit are conveyed, and at the same time the electron beams are irradiated to each front face or back face of the matters through the electron beam irradiation unit in such a manner that the beam irradiating direction is directed to the flat part. Thus, the thin deliverymatters are effectively sterilized during the conveying process.

According to the conveyance method of the present invention, the thin delivery matters are sent into the area where the electron beams are irradiated by the electron beam irradiation unit in such a manner that each front face or back face of the matters is directed substantially perpendicularly to the beam irradiating direction, and the matters are actually irradiated with the beams. Thus, the thin delivery matters can be effectively sterilized.

According to the conveyance method of the present invention, the delivery matters are spread on the flat part of the conveying unit so as not to overlap in the area where the electron beams are irradiated. Therefore, the electron beams can be irradiated evenly to each face of the matters. This makes it possible to reliably sterilize the thin delivery matters.

### INDUSTRIAL APPLICABILITY

As described above, the air supplying system, air sterilizing method, conveyance system, and the conveyance method according to the present invention are useful for facilities like hospitals, factories that produce medical products, food products, semiconductors, or biotechnology related products where highly cleaned air containing no dust particles are needed.

## Claims

1. An air supplying system which sends out air from an air supplying section, and removes dust particles in the air to supply the air containing no dust particles to a specific area, the air supplying system comprising:
an air supply duct which connects the air supply section to the specific area;
a filter which is provided at some point in the air supply duct and removes the dust particles in the air; and
a sterilizing section which includes an electron beam irradiation unit for irradiating electron beams to the air passing through the air supply duct, and which is provided between the air supplying section and the filter, or between the filter and the specific section.

2. The air supplying system according to claim 1, wherein a plurality of the electron beam irradiation units are provided so as to face each other, and the air supply duct is disposed between the two facing electron beam irradiation units.

3. The air supplying system according to claim 1, wherein the air supply duct is provided with a bent,
the filter is disposed near the bent, and
the electron beam irradiation unit is disposed near the bent so that a surface of the filter is irradiated with the electron beams.

4. The air supplying system according to claim 1 or 2, wherein the electron beam irradiation unit is disposed so that electron beams reflected by an internal surface of the air supply duct reach a surface of the filter.

5. The air supplying system according to claim 1, further comprising a fluorescent body which is disposed in the air supply duct and emits light by a stimulus of electron beams emitted from the electron beam irradiation unit.

6. The air supplying system according to claim 5, wherein the fluorescent body is disposed on a bottom of an internal side and internal side faces of the air supply duct.

7. The air supplying system according to any one of claims 1 to 3, 5, and 6, wherein the air supplying section mixes air from outside with air sucked from the specific area, and sends out the mixed air to the air supplying section.

8. The air supplying system according to any one of claims 1 to 3, 5, and 6, wherein the air supplying section takes in air only from the specific area.

9. The air supplying system according to any one of claims 1 to 3, 5, and 6, wherein the air supplying section takes in air only from outside and discharges the air from the specific area to the outside.

10. An air sterilizing method of sterilizing air when air is sent out from an air supplying section and dust particles in the air are removed to supply the air containing no dust particles to a specific area, the air sterilizing method comprising:
removing the dust particles in the air by a filter provided at some point in an air supplying duct which connects the air supply section to the specific area; and
irradiating the air passing through the air supply duct with electron beams between the air supplying section and the filter, or between the filter and the specific area.

11. The air sterilizing method according to claim 10, further comprising irradiating the air in the air supply duct with the electron beams from positions opposite to each other where the air supply duct is disposed therebetween.

12. The air sterilizing method according to claim 10, wherein the air supply duct is provided with a bent, the filter is disposed near the bent, and the electron beams are irradiated to a surface of the filter.

13. The air sterilizing method according to claim 10, further comprising letting the electron beams reflected by an internal surface of the air supply duct, into a surface of the filter.

14. The air sterilizing method according to claim 10, wherein a fluorescent body which emits light by a stimulus of the electron beams, is disposed in the air supply duct, and the electron beams are irradiated to the fluorescent body.

15. The air sterilizing method according to claim 14, wherein the fluorescent body is disposed on a bottom of an internal side and internal side faces of the air supply duct.

16. A conveyance system comprising:
a conveying unit which includes a conveyor and conveys delivery matters such as mails or sealed letters; and
an electron beam irradiation unit which is disposed near the conveying unit and irradiates electron beams to the delivery matters during a conveying process of the matters.

17. A conveyance system comprising:
a conveying unit which includes a flat part formed of a belt part, a roller part, a plate shaped part, and other parts, and conveys thin delivery matters including thin-structured mails and sealed letters with each front face or back face of the matters down on the flat part; and
an electron beam irradiation unit which is disposed in such a manner that a direction of irradiating electron beams is substantially perpendicular to the flat part, and irradiates the electron beams to the thin delivery matters during a conveying process of the matters.

18. A conveyance system comprising:
an electron beam irradiation unit which irradiates electron beams;
a conveying unit which conveys thin delivery matters including thin-structured mails and sealed letters; and
a delivery matter sending unit which sends the delivery matters into an area where the electron beams are irradiated by the electron beam irradiation unit, in such a manner that each front face or back face of the matters is directed substantially perpendicularly to a direction ofirradiating the electron beams.

19. The conveyance system according to any one of claims 16 to 18, further including a pair of electron beam irradiation units which are provided opposite to each other in a vertical direction or a horizontal direction with respect to the conveying unit.

20. The conveyance system according to any one of claims 16 to 18, wherein the conveying unit further includes a delivery matter spreading unit which spreads the delivery matters so that the matters will not overlap in an area where the electron beams are irradiated.

21. A conveyance method comprising steps of:
conveying thin delivery matters including thin-structured mails and sealed letters with each front face or back face of the matters down on a flat part that is formed of a belt part, a roller part, a plate shaped part, and other parts; and
irradiating electron beams substantially perpendicularly to each front face or back face of the thin delivery matters during a conveying process thereof by an electron beam irradiation unit disposed in such a manner that a direction of irradiating the electron beams is substantially perpendicular to the flat part.

22. A conveyance method comprising steps of:
conveying delivery matters including thin-structured mails and sealed letters; and
sending the delivery matters into an area where electron beams are irradiated by an electron beam irradiation unit, in such a manner that each front face or back face of the matters is directed substantially perpendicularly to a direction of irradiating the electron beams.

23. The conveyance method according to claim 21 or 22, further comprising a step of spreading the delivery matters over the flat part so that the matters will not overlap in the are where the electron beams are irradiated.
